# EUROPÄISCHE PATENTSCHRIFT

(11) **EP 0 765 854 B1**
(45) Veröffentlichungstag und Bekanntmachung des Hinweises auf die Patenterteilung: **10.04.2002**
(21) Anmeldenummer: 96115457.2
(22) Anmeldetag: 26.09.1996
(51) Int. Cl.: C07C 51/44, C07C 57/04

(54) **Verfahren zur Reinigung einer Rektifikationskolonne**
Process for the cleaning of a rectification column
Procédé de purification d'une colonne de rectification

(30) Priorität: 28.09.1995 DE 19536179
(43) Veröffentlichungstag der Anmeldung: 02.04.1997
(73) Patentinhaber: BASF AKTIENGESELLSCHAFT, 67056 Ludwigshafen (DE)
(72) Erfinder: Hammon, Ulrich, Dr., 68165 Mannheim (DE); Schliephake, Volker, Dr., 67105 Schifferstadt (DE); Pies, Wolfgang, Dr., 67227 Frankenthal (DE); Rauh, Ulrich, 67117 Limburgerhof (DE)
(74) Vertreter: Isenbruck, Günter, Dr.

(56) Entgegenhaltungen:
- EP-A- 0 648 732
- US-A- 3 470 238

## Beschreibung

Die Erfindung betrifft ein Verfahren zur Reinigung einer Rektifikationskolonne, worin ungesättigte Carbonsäuren aus Lösungsmitteln, in denen diese nach der Synthesereaktion absorbiert wurden, rektifikativ abgetrennt wurden.

Ungesättigte Carbonsäuren des Typs Acrylsäure bzw. Methacrylsäure werden derzeit vornehmlich durch heterogen katalysierte Gasphasenoxidation der entsprechenden Alkene, Alkane oder der ungesättigten Aldehyde industriell hergestellt. Verschiedene derzeit in Anwendung befindliche Verfahren unterscheiden sich vornehmlich in der Art der Oxidationsführung und in der Verfahrenstechnik der Abtrennung der zwangsläufig im Verfahren mitgebildeten Nebenkomponenten (DE-A 19 62 431, DE-A 29 43 707). In der Synthese unterscheiden sich die einzelnen Verfahren darin, ob das Reaktionsgas im Kreis geführt wird und welcher Art das beigefügte Inertgas ist. In der Aufarbeitung liegt der Unterschied vornehmlich darin, ob das Wertprodukt, das heiß und gasförmig aus den Reaktoren kommt, in einem tief- oder hochsiedenden Lösungsmittel absorbiert wird. All diesen Verfahren ist gemeinsam, daß das Wertprodukt am Ende destillativ vom absorbierenden Lösungsmittel getrennt werden muß. Diese Abtrennung erfolgt in der Regel destillativ bzw. rektifikativ.

Zur Vermeidung von Polymerisationen während der Destillation bzw. Rektifikation werden Stabilisatoren, wie z.B. Phenothiazin (PTZ), Hydrochinonmethylether (MeHQ) oder Hydrochinon (HQ) eingesetzt. Dennoch treten nach längeren Laufzeiten in der Rektifikationskolonne Polymerisatbildungen auf, die zur regelmäßigen Abstellung und zu einer aufwendigen Reinigung der Anlage zwingen. Diese Reinigung kann in bekannter Weise mechanisch oder thermisch oxidativ erfolgen. Dies ist jedoch sehr zeitaufwendig.

Der Erfindung lag daher die Aufgabe zugrunde, ein Verfahren zu schaffen, mit dem die Polymerisate in einfacher und zuverlässiger Weise und ohne großen Zeitaufwand entfernt werden können.

Erfindungsgemäß wird dies dadurch erreicht, daß die Rektifikation unterbrochen und die Rektifikationskolonne zur Entfernung von Polymerisat-Niederschlägen mit wäßriger NaOH- oder KOH Lösung gespült wird. Dabei weist die NaOH- oder KOH Lösung eine Konzentration von 0,01 bis 30 Gew.-%, vorzugsweise 0,5 bis 10 Gew.-% auf. Gemäß einer vorteilhaften Weiterbildung der Erfindung wird der NaOH - oder KOH Lösung ein im wesentlichen neutrales Alkali- oder Erdalkalisalz im Verhältnis von >0:1 bis 2:1 (Gewichtsverhältnis von Neutralsalz zu Hydroxid) zugesetzt. Hierfür eignen sich besonders die den basischen Verbindungen entsprechenden Sulfate, Acetate, Oxalate, Carbonate, Hydrogensulfate, Hydrogencarbonate oder andere Salze des Natriums oder Kaliums. Durch einen solchen Zusatz läßt sich das Lösungsverhalten der basischen Lösung weiter verbessern.

Die Temperaturen bei denen die erfindungsgemäße Spülung durchgeführt wird, werden vom Siedepunkt der eingesetzten Lösungsmittel bestimmt, da für alle vorgenannten Lösungsmittel gilt, daß das Lösungsvermögen mit steigender Temperatur zunimmt. Die optimale Temperatur für die wäßrigen Systeme der Alkali- und Erdalkalihydroxide liegt bei 80°C bis ca. 115°C bei Normaldruck, vorzugsweise bei 90°C bis 110°C. Für die beschriebenen Amide liegt die optimale Einsatztemperatur jeweils 10 bis 1 °C unterhalb des Siedepunktes dieser Substanzen.

Das erfindungsgemäße Verfahren kann sowohl in regelmäßigen Zeitabständen wie auch nach Feststellung einer bestimmten Niederschlagsbildung durchgeführt werden. In jedem Fall wird einerseits eine nur geringere Unterbrechungsdauer des Destillationsverfahrens sowie vor allem eine erheblich einfachere und vollständigere Entfernung der Polymerisat-Niederschläge erreicht.

Weitere Einzelheiten und Vorteile der Erfindung können den im folgenden beschriebenen Versuchsbeispielen entnommen werden.

Selbstverständlich kann das erfindungsgemäße Verfahren ganz generell bei der rektifikativen Abtrennung von ungesättigten Carbonsäuren angewendet werden, d.h. zum Beispiel auch bei der Reindestillation von Rohmethacrylsäure zu Reinmethacrylsäure. Als besonders vorteilhaft erweist es sich, das erfindungsgemäße Verfahren präventiv anzuwenden, d.h. zwischenzeitig, bevor eine sichtbare Niederschlagsbildung erfolgt ist. Auf diese Art und Weise wird eine Niederschlagsbildung faktisch im Keim erstickt.

Bei den Laborversuchen ergab sich hierbei folgendes.

### Versuch 1 (Referenzversuch, gemäß dem Stand der Technik)

In einer Glaskolonne (innerer Durchmesser 8 cm, 40 Glockenböden) wurde ein Diphyl/Acrylsäuregemisch destilliert. Dieses Gemisch wurde einer Produktionsanlage entnommen, die nach einem Verfahren arbeitet, wie es in der DE 21 36 396 beschrieben ist.

Der Rücklauf wurde mit Phenothiazin stabilisiert. Nach 238 Stunden staute die Kolonne aufgrund von Polymerisatbildung. Die Kolonne wurde abgestellt und zunächst mit heißem Wasser sechs Stunden lang gespült. Ergebnis: Das Polymerisat wurde nicht entfernt. Es mußte nachträglich mechanisch entfernt werden. Der Zeitaufwand für die Reinigung betrug insgesamt einen kompletten Arbeitstag.

### Versuch 2 (erfindungsgemäße Laugenspülung)

Vorgehen wie bei Versuch 1, Stauerscheinung nach 212 Stunden. Durch Spülung mit einer 5 Gew.-%igen wäßrigen NaOH-Lösung bei einer Temperatur von 92°C war das Polymerisat nach 6 Stunden Spülung vollständig aufgelöst.

### Versuch 3 (erfindungsgemäße Lauge/Sulfat-Spülung)

Vorgehen wie bei Versuch 2, Stauerscheinungen nach 254 Stunden. Durch Spülung mit einer 5 Gew.-% NaOH und 5 Gew.-% Na₂SO₄ enthaltenden wäßrigen Lösung bei einer Temperatur von 92°C konnte bereits nach einer Spülzeit von 5 Stunden die vollständige Entfernung des Polymerisats festgestellt werden.

### Betriebsversuche

Die im Labor gewonnenen Erkenntnissen wurde danach in einer technischen Anlage eingesetzt. Die entsprechenden Versuche wurden dabei in einer Acrylsäureanlage, durchgeführt, die nach einem Verfahren arbeitet, wie es in der DE 21 36 396 beschrieben ist. Dieses Verfahren läßt sich wie folgt kurz charakterisieren:
* Heterogen -katalytische Gasphasenoxidation von Propylen und/oder Acrolein zu Acrylsäure
* Abtrennung der Acrylsäure und eines Teils der Nebenkomponenten aus dem Reaktionsgas durch Gegenstromabsorption mit einem hochsiedenden Lösungsmittel
* Abtrennung einer ersten Fraktion von leicht- und mittelsiedenden Komponenten durch Gegenstromdesorption
* Destillative Abtrennung der Acrylsäure und der leicht- und mittelsiedenden Nebenkomponenten vom hochsiedenden Lösungsmittel. Das Lösungsmittel wird wieder in die Absorptionskolonne rezirkuliert.

Die am Ende dieser Aufarbeitung angeordnete Reinkolonne hat einen Seitenabzug für die gereinigte Roh-Acrylsäure. Um das Auftreten eines Staus durch Polymerisatbildung zu verhindern und eine insgesamt sehr lange Betriebszeit zu erreichen, wurde die Kolonne nach einem Zeitraum von 10 bis 70 Tagen, vorzugsweise von 20 bis 50 Tagen Fahrzeit zum Zwecke der Reinigung nach dem erfindungsgemäßen Verfahren kurzzeitig abgeschaltet. Dies gewährleistete einen sehr langdauernden, sicheren Betrieb der Kolonne. Der Zeitpunkt der Abstellung wurde in Abhängigkeit von Durchsatz und weiteren Parametern festgelegt.

Eine erfindungsgemäße Spülung mit einer 10 Gew.-% igen wäßrigen NaOH-Lösung bei einer Temperatur von 92°C konnte in 8 Stunden abgeschlossen werden.

Noch schneller konnte eine Spülung beendet werden, die mit einer 10 Gew.-%igen wäßrigen NaOH-Lösung, der 5 Gew.-% Natriumsulfat (auf die NaOH-Lösung bezogen) zugesetzt worden waren, und die bei einer Temperatur von 92°C durchgeführt wurde. Hierfür wurden nur 7 Stunden Spülzeit benötigt.

## Patentansprüche

1. Verfahren zur Reinigung einer Rektifikationskolonne, worin ungesättigte Carbonsäuren aus Lösungsmitteln, in denen diese nach der Synthesereaktion absorbiert wurden, rektifikativ abgetrennt wurden, **dadurch gekennzeichnet, daß** die Rektifikation unterbrochen und die Rektifikationskolonne zur Entfernung der Polymerisat-Niederschläge mit wässriger KOH- oder wässriger NaOH-Lösung gespült wird.

2. Verfahren nach Anspruch 1, **dadurch gekennzeichnet, daß** die Unterbrechung in regelmäßigen Zeitabständen erfolgt.

3. Verfahren nach Anspruch 1 oder 2, **dadurch gekennzeichnet, daß** die wäßrige NaOH- oder KOH- Lösung eine Konzentration von 0,01 bis 30 Gew.-%, vorzugsweise 0,5 bis 10 Gew.-%, aufweist.

4. Verfahren nach einem der vorhergehenden Ansprüche, **dadurch gekennzeichnet, daß** der wäßrigen NaOH- oder KOH- Lösung ein neutrales Alkali- oder Erdalkalisalz im Verhältnis von >0:1 bis 2:1 (Gewichtsverhältnis von Neutralsalz zu Hydroxid) zugesetzt wird.

5. Verfahren nach Anspruch 4, **dadurch gekennzeichnet, daß** als neutrale Salze die Sulfate, Acetate, Oxalate, Carbonate oder andere Neutralsalze des Natriums oder des Kaliums zugesetzt werden.

6. Verfahren nach einem der vorhergehenden Ansprüche, **dadurch gekennzeichnet, daß** die Temperatur bei der Spülung 80°C bis 115°C, vorzugsweise 90°C bis 110°C bei Normaldruck beträgt.

## Claims

1. A process for cleaning a rectification column in which unsaturated carboxylic acids were separated by rectification from solvents in which they were absorbed after the synthesis reaction, which comprises interrupting the rectification and flushing the rectification column with an aqueous NaOH solution to remove polymer deposits.

2. A process as claimed in claim 1, wherein the interrupting is effected at regular time intervals.

3. A process as claimed in claim 1 or 2, wherein the aqueous NaOH solution has a concentration of from 0.01 to 30 % by weight, preferably from 0.5 to 10 % by weight.

4. A process as claimed in any of the preceding claims, wherein a neutral alkali metal or alkaline earth metal salt is added to the aqueous NaOH solution in a ratio of from > 0:1 to 2:1 (weight ratio of neutral salt to hydroxide).

5. A process as claimed in claim 4, wherein the neutral salts added are the sulfates, acetates, oxalates, carbonates or other neutral salts of sodium.

6. A process as claimed in any of the preceding claims, wherein flushing is carried out at from 80°C to 115°C, preferably from 90°C to 110°C, and at atmospheric pressure.

## Revendications

1. Procédé de purification d'une colonne de rectification dans laquelle on a séparé par rectification des acides carboniques d'avec des solvants ayant absorbés ces acides après la réaction de synthèse, **caractérisé en ce que** l'on interrompe la rectification et que l'on rince la colonne de rectification avec une solution aqueuse de NaOH afin d'éliminer les dépôts de polymères.

2. Procédé selon la revendication 1, **caractérisé en ce que** l'on procède à une interruption à des intervalles de temps réguliers.

3. Procédé selon la revendication 1 ou 2, **caractérisé en ce que** la solution aqueuse de NaOH présente une concentration comprise entre 0,01% et 30% en poids, de préférence entre 0,5% et 10% en poids.

4. Procédé selon l'une quelconque des revendications précédentes, **caractérisé en ce que** l'on ajoute à la solution aqueuse de NaOH, un sel alcalino-terreux ou alcalin neutre, dans une proportion comprise entre supérieur à 0:1 et 2:1 (proportion pondérale de sel neutre et d'hydroxyde).

5. Procédé selon la revendication 4, **caractérisé en ce que** l'on ajoute, en tant que sels neutres, des sulfates, des acétates, des oxalates, des carbonates ou d'autres sels neutres du sodium.

6. Procédé selon une quelconque des revendications précédentes, **caractérisé en ce que** l'on réalise le rinçage à une température comprise entre 80°C à 115°C, de préférence entre 90°C et 110°C, et à pression atmosphérique normale.
